# EUROPEAN PATENT APPLICATION

(11) **EP 4 169 431 A1**
(43) Date of publication of application: **26.04.2023**
(21) Application number: 21204436.6
(22) Date of filing: 25.10.2021
(51) Int. Cl.: A61B 1/00, A61B 1/005, A61B 1/05

(54) **AN ENDOSCOPE**

(71) Applicant: Ambu A/S, 2750 Ballerup (DK)
(72) Inventor: HANSEN, Frederik Clausager Vemb, 2400 Copenhagen NV (DK); SØRENSEN, Morten, 2750 Ballerup (DK)
(74) Representative: COPA Copenhagen Patents

(57) **Abstract**

An endoscope comprising a bending section, a steering wire, a distal tip part including an exterior housing including a circumferentially extending side wall with a proximal opening, a camera assembly positioned in an interior cavity of the exterior housing, and an interior housing part positioned in the exterior housing, wherein a distal segment of the at least one steering wire is fixed to a wire portion of the interior housing part so that manipulation of the control device causes bending of the bending section.

## Description

### TECHNICAL FIELD

The present disclosure relates to a steerable endoscope having a bending section and a distal tip part, and a method of assembling such an endoscope.

### BACKGROUND

Endoscopes are well known for visually inspecting inaccessible places, such as human body cavities. Typically, the endoscope comprises an elongated insertion cord with a handle at the proximal end, as seen from the operator, and visual inspection means, such as a built-in camera, at the distal end of the elongated insertion cord, and an insertion tube extending between the handle and the visual inspection means. This definition of the terms "distal" and "proximal", i.e. proximal being the end closest to the operator and distal being the end remote from the operator, as used herein for endoscopes in general, is adhered to in the present disclosure. Endoscopes in the present context also cover laryngoscopes and endotracheal tubes provided with a camera for surveillance of correct positioning.

As the name indicates, endoscopes are used for seeing inside things, such as lungs or other human body cavities of a patient. Modern endoscopes are therefore typically equipped with a light source and a vision receptor including a vision sensor, such as a camera or an image sensor. Provided that sufficient light is present, it is possible for the operator to see, where the endoscope is steered and to set the target of interest once the tip has been advanced thereto. This therefore normally requires illumination of the area in front of the distal tip part of the endoscope, in particular the field of vision of the camera(s). The light source, such as a light-emitting diode or an optical fibre, may provide illumination.

Electrical wiring for the camera and other electronics, such as LED lighting accommodated in the tip part at the distal end, runs along the inside of the elongated insertion cord from the handle to the tip part. Instead of using cameras, endoscopes may also be fibre-optic, in which case the optical fibres run along the inside of the elongated insertion cord to the tip part. For some applications, a working or suction channel may run along the inside of the insertion cord from the handle to the tip part, e.g. allowing liquid to be removed from the body cavity, allowing for injection of fluid into the body cavity, or allowing for insertion of surgical instruments or the like, into the body cavity. The suction channel may be connected to a suction connector, typically positioned at a handle at the proximal end of the insertion cord. For other applications, the working or suction channel may be omitted.

In order to be able to manoeuvre the endoscope inside the body cavity, the distal end of the endoscope may comprise a bending section with increased flexibility, e.g. an articulated tip part allowing the operator to bend this section. Typically, this is done by tensioning or slacking steering wires also running along the inside of the elongated insertion cord from the articulated tip part to a control mechanism of the handle. Furthermore, a working channel may run along the inside of the insertion cord from the handle to the tip, e.g. allowing liquid to be removed from the body cavity or allowing for the insertion of surgical instruments or the like into the body cavity.

A general desire in the field of endoscopy is to miniaturise the insertion cord of the endoscope and thus the tip part, as this may open new fields of application. In certain types of endoscopy procedures, such as ureteroscopy, an endoscope with an outer diameter of no more than 3 mm is required. Alternatively, such miniaturising may allow for the provision of a larger working channel diameter, thus expanding the range of tools that can pass available, while maintaining the same outer diameter. However, it has been found that especially for small endoscopes with an outer diameter around or below 3 mm, such as for instance a ureteroscope, fastening of the steering wires at the distal end of the bending section is difficult due to the limited space and due to the difficulties of gluing to the material of the bending section, which in a single-use endoscope typically is an integrally moulded and flexible part.

A further general desire in the field of endoscopy is to electrically insulate the insertion cord, and thus especially the tip part, from the outside, so as to mitigate the risk of an insulation breakdown and a resulting excessive leakage current.

Another general desire in the field of endoscopy is to provide a distal tip part, which is liquid-sealed, so as to mitigate liquid ingress into the tip part, and specifically into any electrical or optical components of the tip part.

### SUMMARY

On this background, it may be seen as an object of the present disclosure to provide an improved tip part for an endoscope alleviating or meeting at least some of the above-mentioned desires.

One or more of these objects may be met by aspects of the present disclosure as described in the following.

A first aspect of this disclosure relates to an endoscope for visually inspecting inaccessible places, such as human body cavities, the endoscope extending along a proximal-distal axis from a handle to a distal tip part, the endoscope comprising:
- the distal tip part including:
   ∘ an exterior housing including a circumferentially extending side wall with a proximal opening, the circumferentially extending side wall defining an interior cavity preferably with a gas volume,
   ∘ a camera assembly including an image sensor, the camera assembly being positioned in the interior cavity of the exterior housing preferably so that the gas volume envelops at least part of the camera assembly, and
   ∘ an interior housing part being positioned in the proximal opening of the exterior housing and being a prefabricated component formed separately from the exterior housing, the interior housing part including:
      ▪ a distal portion with a circumferentially extending closure surface closing the interior cavity of the exterior housing preferably so as to liquid-seal the gas volume of the interior cavity, and
      ▪ a proximal portion with a circumferentially extending attachment surface, the proximal portion being distanced in a proximal direction from the exterior housing;
- a bending section including a proximal end segment, a distal end segment, and a number of intermediate segments arranged between the proximal end segment and the distal end segment, the distal end segment being attached, preferably by adhering, to the attachment surface of the interior housing part, the bending section being a prefabricated component formed separately from the exterior housing and the interior housing part;
- an insertion tube for insertion into a patient attached to the proximal end segment of the bending section;
- the handle for gripping by an operator, the handle being attached to the insertion tube opposite of the bending section, the handle comprising a control device; and
- at least one steering wire attached to the control device of the handle and running through the bending section and the insertion tube;
wherein a distal segment of the at least one steering wire is fixed to a wire portion of the interior housing part so that manipulation of the control device causes bending of the bending section.

In conventional endoscopes, the steering wire(s) is/are attached to the distal end segment of the bending section. However, the inventors of the present disclosure have found that the steering wire(s) may instead be fixed to an interior housing part of the distal tip part. Such an arrangement may provide several advantages. The material of the interior housing part is less restricted than the material of the bending section. The bending section is a high-performance part coping with relatively high tensioning forces by the steering wire(s) and straining during bending to large angles, in some cases 210 degrees or more. Therefore, the bending section is typically made of a flexible polymer, such as polyoxymethylene (POM), which is advantageous for these functions. However, the conventional materials of the bending section typically exhibit poor adhesion by adhesives and are typically opaque. Therefore, an advantage of fixing the steering wire(s) to the interior housing part is that it may be easier to adhere the steering wire(s) to the interior housing part, as the material of the interior housing part can be suitably chosen, e.g. polycarbonate. Further, an adhesive curable by ultraviolet light (UV) may be employed, as the interior housing part can be made transparent to UV light. Accordingly, the attachment of the interior housing part and the steering wires may be made stronger and have increased manufacturability.

Another advantage may be a bending section with a smaller outer diameter. Since the bending section may accommodate dedicated passages for data/power cables for the camera assembly, a working channel for insertion of tools, and dedicated steering wire lumens for retaining the steering wire(s), it is difficult also to securely fix the steering wire(s) to the bending section. As mentioned, this is especially the case when adhering the steering wire(s) to a bending section made of POM. Thus, by fixing the steering wire(s) to the interior housing part instead of the bending section, the steering wire lumens can be made open towards the working channel passage. Accordingly, the outer diameter of the bending section can be reduced.

In the context of the present disclosure, the proximal-distal axis of the endoscope may extend along a central line of the insertion cord, the bending section, and the tip part. The terms "proximal" and "distal" are understood in reference to the closeness to an operator using the endoscope. For example, the handle defines the proximal end of the endoscope, while the distal tip part defines the distal end of the endoscope. Further, the terms "circumferentially extending", "radially extending", and "longitudinal extending" are understood with respect to the proximal-distal axis of the endoscope.

Additionally or alternatively, the endoscope may comprise one or more cables running through the insertion tube and electrically connecting the electrical circuit of the camera assembly with the handle. The one or more cables may run in a passage of the bending section. The one or more cables may pass through one or more through-holes of the interior housing part. In particular, the one or more cables may pass through a cable passage of the proximal portion and a cable hole of the distal portion of the interior housing part. In the context of the present disclosure, the word "cable" is an insulated wire, and the word "cables" denotes a plurality of insulated wires, which may or may not be enclosed in a sheath.

Additionally or alternatively, the exterior surface of the side wall of the exterior housing may be cylindrically shaped. An exterior diameter of the exterior surface of the side wall may define the exterior diameter of the distal tip part. The exterior diameter may be at most 3.5 mm or preferably at most 3.0 mm.

Additionally or alternatively, the exterior housing may comprise a working channel passage that may extend between a proximal opening in the proximal opening and a distal opening at the distal end of the exterior housing. The working channel passage may be separated from the interior cavity by an interior wall.

Additionally or alternatively, the interior housing part may be integrally formed as a monolithic component (e.g. moulded in one piece of material), preferably by injection moulding.

Additionally or alternatively, the exterior housing may be integrally formed as a monolithic component, preferably by injection moulding. The exterior housing may be moulded from a single material (e.g. moulded in one piece of material). Alternatively, the exterior housing may be moulded from two different materials (e.g. a transparent and an opaque material) in a single piece, by a two-component injection moulding process, for example as described in EP 3 539 449 A1.

Additionally or alternatively, the bending section may be integrally formed as a monolithic component (e.g. moulded in one piece of material), preferably by injection moulding.

Such a bending section may ease the assembly as the bending section itself does not require assembly.

Additionally or alternatively, the bending section may comprise or consist essentially of a polymer, preferably flexible polymer, such as polyoxymethylene (POM).

Additionally or alternatively, each hinge member between the segments of the bending section may be living hinges.

Additionally or alternatively, the wire portion of the interior housing part may comprise a channel that may accommodate and/or retain the distal segment of the at least one steering wire.

This may be particularly useful during assembly, as the distal segment of the at least one wire can be placed in the channel and allow the interior housing part and at least one steering wire to be handled together without the at least one steering wire becoming loose.

Additionally or alternatively, the distal segment of the at least one steering wire may pass one or more friction-inducing sections of the wire portion that may be configured to frictionally retain the at least one steering wire in the wire portion.

This may provide the advantage of reducing or even eliminating the risk of the distal segment of the at least one steering wire sliding with respect to the wire portion.

Each friction-inducing section of the wire portion may for example be a corner, deformity, or crimp. Preferably, the one or more friction-inducing sections include(s) a plurality of corners around which the distal segment of the at least one steering wire may be bent around to increase a friction force that prevents the distal segment from sliding relative to the wire portion. The one or more friction-inducing sections may include(s) a first pair of corners that may be arranged adjacent to and on opposite sides of the channel, and about which the distal segment of the at least one steering wire bends at an angle, which may be in the range of 45-135 degrees, preferably 70-90 degrees.

Additionally or alternatively, the distal tip part may further comprise a separately formed planar element that may comprise at least one through-hole, preferably two through-holes. The planar element may be arranged between the interior housing part and the bending section. The at least one steering wire may extend through the at least one through-hole, preferably the two through-holes.

Such a planar element may ease the control of the at least one steering wire during assembly and may assist in keeping the at least one steering wire in the correct position during assembly. Furthermore, the planar element may assist in keeping the steering wire in position during full bending of the bending section.

Additionally or alternatively, the planar element may comprise or may preferably consist essentially of a stiffer material than the interior housing part. The material of the planar element may be a metal. The planar element may be formed by a sheet metal forming process, such as punching.

Additionally or alternatively, the planar element may contact a proximal end of the interior housing part.

Additionally or alternatively, the planar element may be a platelike element and/or may be shaped in the form of a disc and/or a crescent.

Additionally or alternatively, the wire portion of the interior housing part and/or the at least one through-hole of the planar element may be configured to bend a distal segment of the at least one steering wire away from the proximal-distal axis by an angle at the respective through-hole. The angle may be at least 2°, preferably at least 5°.

Such an angle may increase the friction between the at least one steering wire and the interior housing part and/or the planar element. Thereby, the maximum load on the at least one steering wire before sliding in relation to the interior housing part is increased.

Additionally or alternatively, an angle between the distal segment of the at least one steering wire and the centre line of the respective through hole may be at least 2°, preferably at least 5°. It is considered that such an angle will increase the maximum pull force on the steering wire.

Additionally or alternatively, a segment of the at least one steering wire extending immediately from the planar element and towards the interior housing part is non-parallel with respect to a centre line of the respective through-hole of the planar element.

Additionally or alternatively, the distal tip part may comprise a cured first adhesive that may adhere the circumferentially extending closure surface to an interior surface of the side wall of the exterior housing, thereby closing the interior cavity of the exterior housing and preferably liquid-sealing the gas volume of the interior cavity.

Additionally or alternatively, the closure surface of the distal portion of the interior housing part and the interior surface of the exterior housing is distanced by a substantially uniform distance about the proximal-distal axis. This distance and the viscosity of the first adhesive is chosen so that capillary forces urge the liquid first adhesive to flow into the gap between closure surface and the interior surface. Further, the distal tip part is arranged so that space significantly expands on the distal side of this gap so that liquid adhesive is kept within the gap until curing and thus prevented from flowing onwards from the gap and into the interior cavity.

Additionally or alternatively, the distal tip part may comprise a cured second adhesive that may adhere the at least one steering wire to the wire portion of the interior housing part, preferably to the channel and/or friction-inducing section(s) of the wire portion.

Additionally or alternatively, the first and second adhesives may preferably be different from each other but may in some embodiments be the same. The first and second adhesives may preferably be cured at different times during assembly, i.e. the first adhesive is cured first, and the second adhesive is cured subsequently. In such a case, a border between the two adhesives may be present and intermixing of the adhesives may be avoided. However, in some embodiments, the adhesives may be cured simultaneously, for instance when the first and second adhesives are the same type of adhesive.

Additionally or alternatively, the distal end segment of the bending section may comprise a circumferentially extending outer wall that may have a proximal-distal extending cut-out. The proximal portion of the interior housing part may comprise one or more locking protrusions radially extending into the cut-out of the outer wall of the bending section so as to lock the rotation of the interior housing part with respect to the distal end segment of the bending section about the proximal-distal axis.

This may be advantageous during assembly, as the orientation of the bending section relative to the interior housing part is locked, thus reducing the risk of erroneous assembly. Furthermore, the attachment strength between the bending section and the interior housing part can be further improved by arranging the cured second adhesive around the locking protrusions in the cut out so as to form an adhesive anchor.

Additionally or alternatively, the at least one steering wire may include a first steering wire and/or a second steering wire.

In some embodiments, the first and second steering wires may be separate from each other. In such embodiments, the distal segment of each steering wire may be attached to the wire portion of the interior housing part.

In other embodiments, the first steering wire may comprise two longitudinal segments extending in parallel along the proximal-distal axis. The first steering wire may comprise the distal segment, which may be arranged between the two longitudinal segments. Additionally or alternatively, the two longitudinal segments may extend from the handle to the interior housing part. The two longitudinal segments of the first steering wire may each comprise an end of the single steering wire. The ends of the first steering wire may be connected or attached directly to the control device of the handle. Additionally or alternatively, the distal segment may connect two longitudinal segments of the first steering wire. The distal segment of the first steering wire may be fixed to the wire portion of the interior housing part.

Additionally or alternatively, the interior housing part may include:
- an intermediate portion that may be arranged between and separating the distal portion of the interior housing part and the proximal portion of the interior housing part, and/or
- a cured adhesive surface that may extend circumferentially and between the proximal opening of the exterior housing and the proximal portion of the interior housing part;
wherein the endoscope may further comprise a bending cover arranged exteriorly of and covering the bending section, preferably covering gaps between segments of the bending section. A distal portion of bending cover may be adhered to the circumferentially extending cured adhesive surface of the interior housing part.

By adhering the bending cover to an already cured adhesive surface, an improved contact is ensured. This is in contrast to conventional endoscopes, where the distal end of the bending cover is typically adhered to the bending section, the resulting connection typically having poor adhesion.

Additionally, the bending cover may be arranged to not overlap the exterior housing. Additionally or alternatively, the bending cover may be arranged proximally of the proximal opening and/or the proximal end of the exterior housing. By arranging the bending cover in such a way, the outer diameter of the distal tip part is kept to a minimum, since the thickness of the bending cover does not enlarge the outer diameter.

A second aspect of this disclosure relates to a method of assembling a distal tip part for an endoscope preferably according to the first aspect. The method comprises the steps of:
- separately providing:
   ∘ an exterior housing including a circumferentially extending side wall with a proximal opening, the circumferentially extending side wall defining an interior cavity preferably with a gas volume;
   ∘ a camera assembly including an image sensor;
   ∘ an interior housing part including:
      ▪ a distal portion with a circumferentially extending closure surface, and
      ▪ a proximal portion with a circumferentially extending attachment surface; and
   ∘ preferably at least one steering wire having a distal segment;
- preferably attaching the distal segment of the at least one steering wire to a wire portion of the interior housing part; and
- assembling the exterior housing, the camera assembly, and the interior housing part;

Additionally or alternatively, the step of assembling the exterior housing, the camera assembly, and the interior housing part may comprise a first set of steps including:
- preferably positioning the exterior housing upright, preferably in a jig, so that the proximal opening faces upwards,
- inserting the camera assembly through the proximal opening and into the interior cavity of the exterior housing, preferably so that the gas volume envelops at least part of the camera assembly, preferably the optical axis of the camera assembly;
- inserting the interior housing part through the proximal opening of the exterior housing so that a substantially uniform gap is formed between the closure surface of distal portion of the interior housing part and an interior surface of the side wall of the exterior housing;
- injecting a first adhesive through the proximal opening so that the first adhesive fills the substantially uniform gap and preferably until a level of the first adhesive reaches the proximal opening of the exterior housing; and
- causing or letting the first adhesive cure thereby forming a sub-assembly in which the interior housing part is adhered to the exterior housing and preferably the gas volume of the interior cavity is liquid sealed by the first adhesive.

Additionally, during the step of inserting the interior housing part through the proximal opening of the exterior housing, the substantially uniform gap is provided by one or more guiding protrusion(s) of the closure surface of the interior housing part. The one or more guiding protrusion(s) contacts or abuts the interior surface of the side wall of the exterior housing when the distal portion of the interior housing part is placed in the exterior housing.

Additionally or alternatively, the step of assembling the exterior housing, the camera assembly, and the interior housing part of the method of the second aspect may further comprise the steps of:
- separately providing:
   ∘ a bending section including a proximal end segment, a distal end segment, and a number of intermediate segments arranged between the proximal end segment and the distal end segment, and
   ∘ a mould including a mould volume and an exterior injection port providing an injection passage to the mould volume;
- inserting the interior housing part and a distal end segment of the bending section into the mould so that the circumference of the sealing portion of the mould expands around the exterior housing and the sealing portion forms a circumferentially extending liquid seal at a proximal end of the exterior housing, and so that the mould volume of the mould envelops a portion of the interior housing part extending from the proximal opening of the exterior housing to the proximal portion, preferably a proximal end, of the interior housing part;
- injecting a second adhesive through the exterior injection port of the mould so that the second adhesive fills the mould volume of the mould; and
- causing or letting the second adhesive cure so that the attachment surface of the interior housing part is adhered to the distal end segment of the bending section and thereby attaching the distal segment of the at least one steering wire to the wire portion of the interior housing part.

Additionally or alternatively, the step of assembling the exterior housing, the camera assembly, and the interior housing part may comprise a second set of steps, which may preferably be performed after the first set of steps. The second set of steps includes:
- separately providing:
   ∘ a bending section including a proximal end segment, a distal end segment, and a number of intermediate segments arranged between the proximal end segment and the distal end segment, and
   ∘ a mould including a mould volume and an exterior injection port providing an injection passage to the mould volume;

- inserting the distal end segment of the bending section and the sub-assembly into the mould so that the sealing portion forms a circumferentially extending liquid seal at a proximal end of the exterior surface of the exterior housing, and so that the mould volume of the mould envelops a portion of the interior housing part extending from the proximal opening of the exterior housing to the proximal portion of the interior housing part;
- injecting a second adhesive through the exterior injection port of the mould so that the second adhesive fills the mould volume of the mould; and
- causing or letting the second adhesive cure so that the attachment surface of the interior housing part is adhered to the distal end segment of the bending section and thereby attaching the distal segment of the at least one steering wire to the wire portion of the interior housing part.

Additionally or alternatively, prior to injecting the second adhesive, the mould volume may comprise, a circumferentially extending gap, which may extend longitudinally between the proximal portion of the interior housing part and the circumferentially extending liquid seal of the mould. The step of injecting the second adhesive may comprise filling said circumferentially extending gap with the second adhesive. The step of causing or letting the second adhesive cure may comprise overmoulding, upon curing, a circumferentially extending cured adhesive surface onto the interior housing part between the proximal opening of the exterior housing and the proximal portion of the interior housing part. The method may further comprise a step of adhering a distal portion of a bending cover to the cured adhesive surface so that the exterior surface of the bending cover is substantially flush with the exterior surface of the exterior housing and preferably so that the bending cover covers the bending section, preferably any gaps between hinge members of the bending section.

Additionally or alternatively, the mould may be a transparent mould, preferably made of silicone, the second adhesive may be curable by ultraviolet light, and the step of causing the second adhesive to cure may include exposing the second adhesive to ultraviolet light through the transparent mould.

A person skilled in the art will appreciate that anyone or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail in the following with regard to the accompanying figures. The figures show one way of implementing the present invention and are not to be construed as being limiting to other possible embodiments falling within the scope of the attached claim set.
Fig. 1 is a schematic side view of an endoscope and illustrating cross-sectional line A-A.
Fig. 2 is a schematic perspective view of a monitor for being connected to the endoscope of Fig. 1.
Fig. 3 is a schematic cross-sectional view of the distal tip part along cross-sectional line A-A as shown in Fig. 1.
Fig. 4a is a schematic perspective view of the distal tip part as cut along line B-B as shown in Fig. 3.
Fig. 4b is a schematic perspective view of the distal tip part as cut along line C-C as shown in Fig. 3.
Fig. 5 is a schematic perspective view of an interior housing part and a steering wire.
Fig. 6 is a schematic side view of the connection between the exterior housing, the interior housing part, and the bending section with hidden lines shown as dashed lines.
Fig. 7a is a schematic perspective view of a step of injecting a first adhesive.
Fig. 7b is a schematic side view of Fig. 7a with hidden lines shown as dashed lines.
Fig. 8 is a schematic cross-sectional view of the distal tip part along cross-sectional line B-B as shown in Fig. 3 after curing of the first adhesive.
Fig. 9 is a perspective view of a step of injecting of a second adhesive with hidden lines shown as dashed lines.
Fig. 10 is a schematic side view of the distal tip part after curing of the second adhesive with hidden lines shown as dashed lines.

### DETAILED DESCRIPTION OF THE INVENTION

In the following figure description, the same reference numbers refer to the same elements and may thus not be described in relation to all figures.

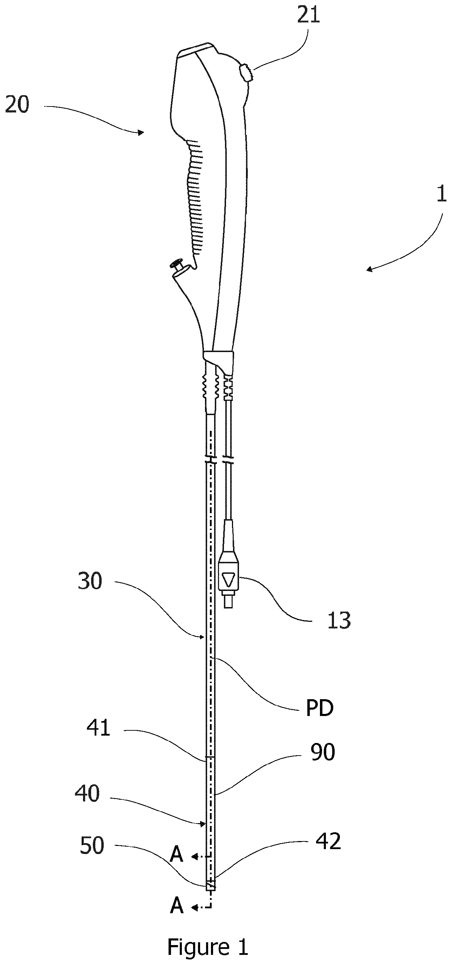

Fig. 1 illustrates an endoscope 1, which is disposable and not intended to be cleaned and reused. The endoscope 1 comprises a distal tip part 50, a handle 20 for gripping with a control device (in the form of a control lever 21), an insertion tube 30, for insertion into a patient, and a bending section 40.

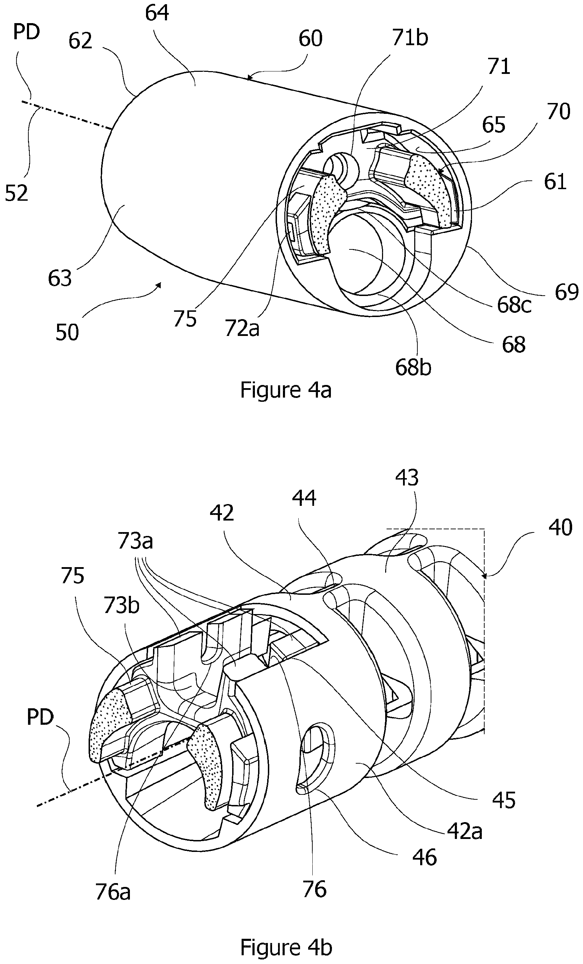

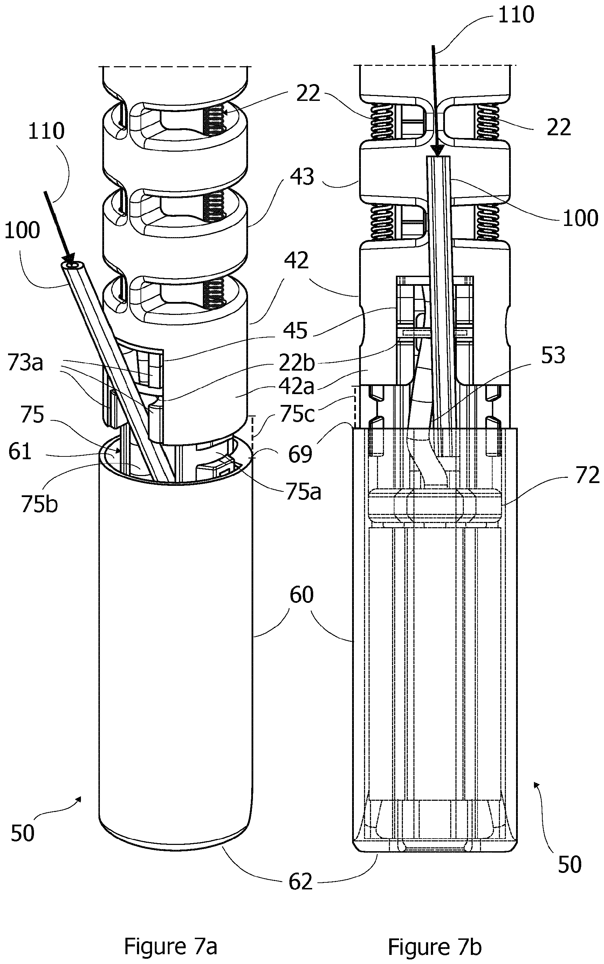

The bending section 40 is integrally formed by injection moulding as a monolithic component preferably made of polyoxymethylene (POM). The bending section 40 comprises articulated segments including a proximal end segment 41, a distal end segment 42, and a number of intermediate segments 43 (as best seen in Figures 7a and 7b) between the proximal end segment 41 and the distal end segment 42. The bending section 40 further comprises living hinge members 44 connecting the segments 41, 42, 43, which is best seen in Figures 4b, and 7a.

Returning to Fig. 1, the insertion tube 30 extends between the handle 20 and the proximal end segment 41 of the bending section 40. The insertion tube 30 has an exterior tubular surface facing the surroundings of the endoscope 1.

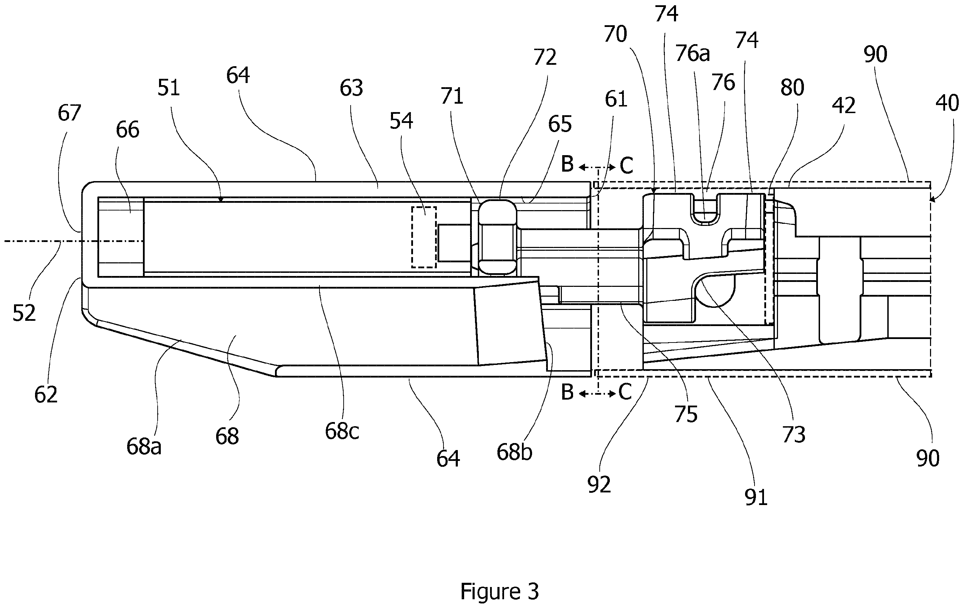

The endoscope 1 of Fig. 1 is shown with a bending cover 90 in the form of a thin outer sleeve. The bending cover 90 covers the segments 41, 42, 43 and hinge members 44 of the bending section 40 thereby providing an additional layer of sealing for the gaps between the segments 41, 42, 43 of the bending section 40. The bending cover 90 also provides a smooth exterior surface for the bending section 40 to improve the comfort of a patient undergoing endoscopy. The position of the bending cover 90 is indicated on Figure 3 with dashed lines but is otherwise omitted in the remaining figures for visualisation purposes.

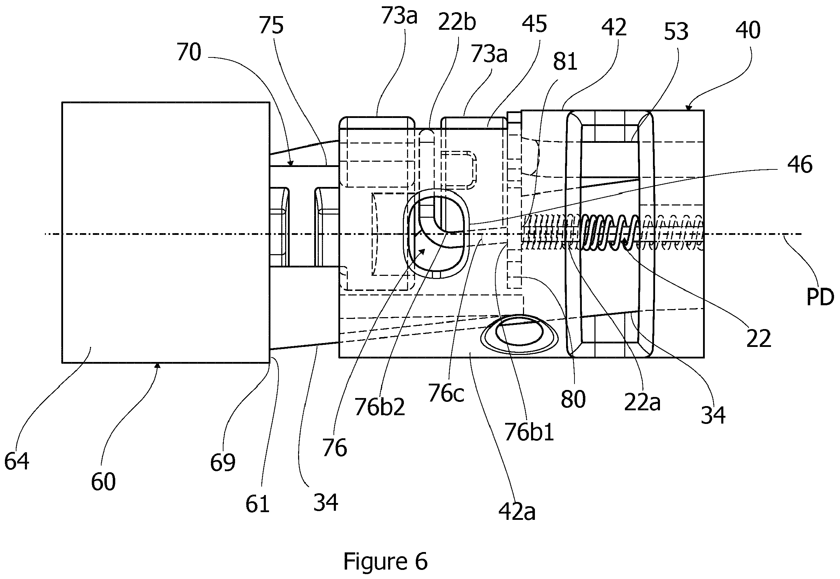

As best seen in Figure 6, a working channel extends inside a working channel tube 34 that runs along the inside of the insertion tube 30 from a working channel opening (not shown) of the handle 20 to a distal tip working channel opening 68 (best seen in Figure 3). The working channel allows liquid or air to be added to and/or removed from the body cavity or allows for insertion of medical tools or surgical instruments into the body cavity.

Returning to Figure 1, the endoscope 1 further has a proximal-distal axis PD that coincides with a central line of the insertion tube 30, the bending section 40, and the distal tip part 50.

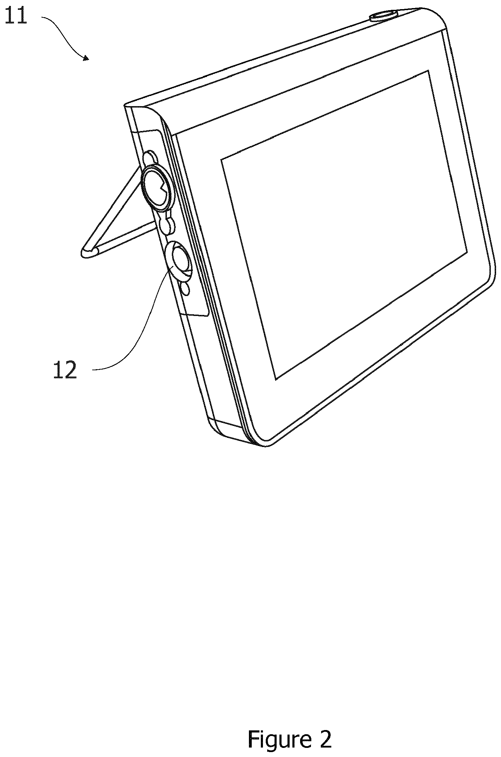

In Fig. 2, a monitor 11 is shown. The monitor 11 comprises a cable socket 12 to which a monitor cable 13 of the endoscope 1 (shown in Fig. 1) can be connected to establish signal communication between the camera assembly 51 (shown in Figure 3) of the endoscope 1 and the monitor 11 via the cables 53 and the circuit (not shown) of the handle. The monitor 11 display images and/or video captured by the camera of the endoscope 1 thus allowing an operator to "see" the body cavity through the camera of the endoscope 1.

Turning now to Figure 3, the distal tip part 50 comprises an exterior housing 60, a camera assembly 51, and an interior housing part 70.

The exterior housing 60 comprises a distal end 62 with a window 67, a proximal end 69, a circumferentially extending side wall 63 with a proximal opening 61 at the proximal end 69. The circumferentially extending side wall 63 defines an interior cavity 66 with a gas volume. The exterior housing 60 has an exterior surface 64 facing the exterior of the endoscope and an interior surface 65 facing the interior cavity 66. The exterior housing 60 further comprises a working channel passage 68 extending between a proximal opening 68b in the proximal opening 61 and a distal opening 68a at the distal end 62 of the exterior housing 60. The working channel passage 68 is separated from the interior cavity 66 by an interior wall 68c. A working channel tube 34 (not shown in Figure 3) is connected to the proximal opening 68b of the working channel passage 68, as indicated in Figure 6. The exterior surface 64 of the side wall 63 of the exterior housing is cylindrically shaped. The exterior diameter of the side wall 63 is 3.0 mm and defines the maximum outer diameter of the distal tip part 50, the bending section 40, and the insertion tube 30.

The camera assembly 51 is positioned in the interior cavity of the exterior housing 60 of the distal tip part 50. The camera assembly 51 is in signal communication with a circuit (not shown) of the handle 20 via data and power cables 53 (as best seen in Figures 6, 7b and 10). The camera assembly comprises an image sensor 54 (as indicated in Figure 3) viewing in an optical axis 52 through a lens barrel (not shown). The camera assembly 51 is arranged so that the optical axis 52 passes through the window 67 at the distal end 62 of the exterior housing 60. The lens barrel comprises several lenses enclosed in an opaque barrel typically provided by a polymer cylinder with an opaque, e.g. black, coating or paint provided circumferentially around on the outside of the cylinder. The lenses optimise the view of the image sensor 54. The lens barrel typically forms an optical barrier which reduces stray light entering the image sensor 54.

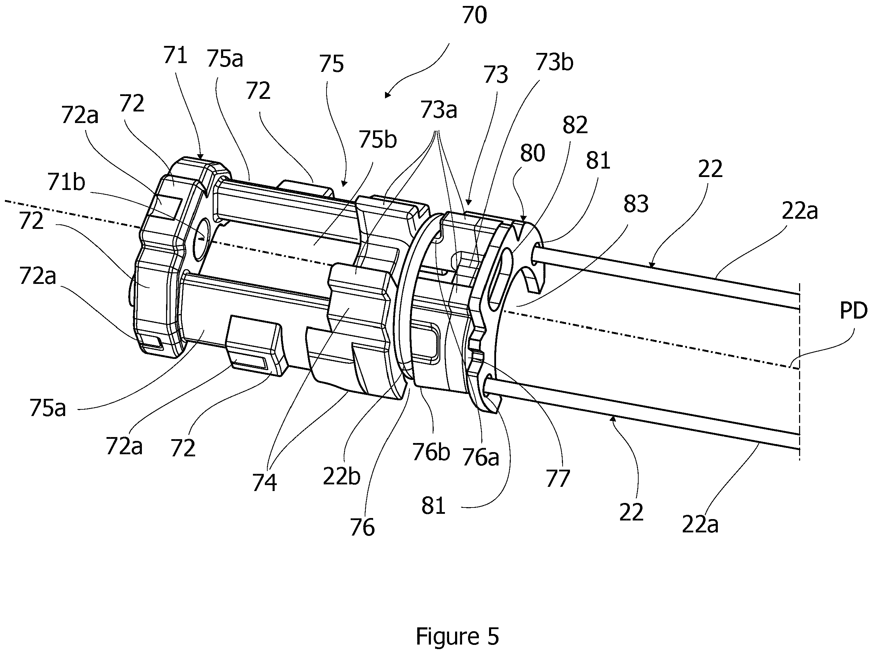

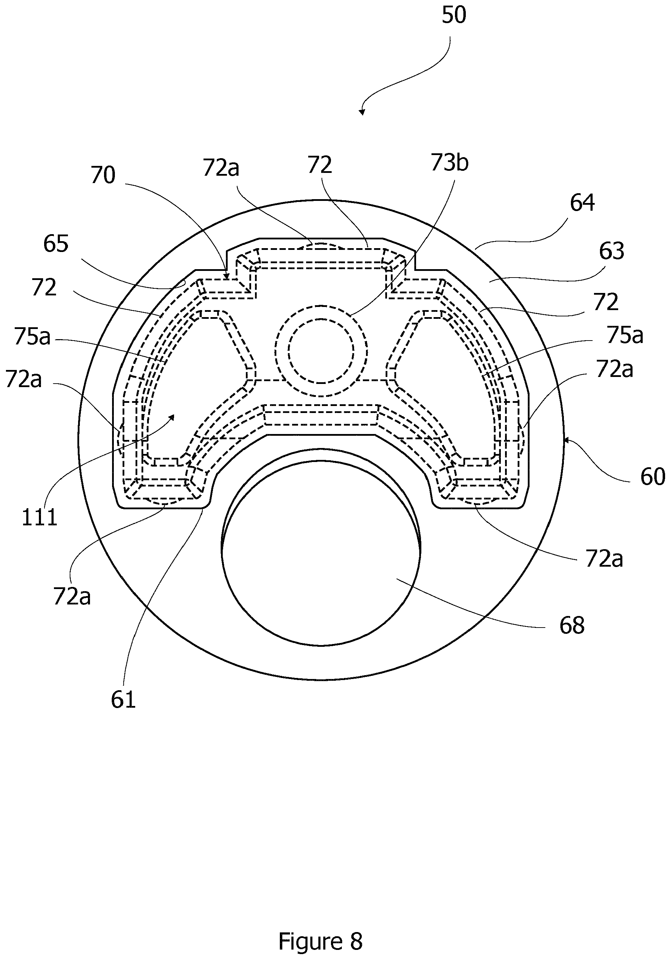

The interior housing part 70 is a prefabricated component formed separately from the exterior housing 60, as best seen in Figure 5. As seen in Figures 3 and 4a, the interior housing part 70 is positioned in the proximal opening 61 of the exterior housing 60. As can be best seen in Figure 5, the interior housing part 70 includes a distal portion 71, a proximal portion 73, and an intermediate portion 75 between the distal portion 71 and the proximal portion 73. The distal portion 71 has a circumferentially extending closure surface 72 closing the interior cavity 66 of the exterior housing 60 as shown in Figures 3 and 4a. A circumferentially extending uniform gap is formed between the closure surface 72 and the interior surface 65, which is filled with a first adhesive, as described in connection with Figures 7a, 7b and 8 to seal the gas volume of the interior cavity 66. The proximal portion 73 has a circumferentially extending attachment surface 74. The proximal portion 73 is distanced in a proximal direction along the proximal-distal axis PD from the proximal end 69 of the exterior housing 60 as best seen in Figure 3. As best seen in Figure 4a, the distal portion 71 of the interior housing part 70 comprises a cable hole 71b for the cable 53 (the cable 53 is omitted in Figure 4a but can be seen in Figure 7b).

Turning to Figure 4b, the proximal portion 73 is positioned in the distal end segment 42 of the bending section 40. The distal end segment 42 of the bending section 40 comprises a circumferentially extending outer wall 42a with a proximal-distal extending cut out 45. The proximal portion 73 of the interior housing part 70 comprises four locking protrusions 73a radially extending into the cut-out 45 of the outer wall 42a of the bending section 40 and locking the rotation of the interior housing part 70 with respect to the distal end segment 43 of the bending section about the proximal-distal axis PD. The proximal portion 73 further comprises a cable passage 73b for the cable 53 (omitted in Figure 4b but can be seen in Figure 7b).

Turning to Figure 5, the interior housing part 70 is shown together with a single steering wire 22 of the endoscope. The single steering wire 22 includes two longitudinal segments 22a extending in parallel along the proximal-distal axis PD. The ends of the longitudinal segments 22a of the steering wire 22 are attached to the control lever 21 of the handle 20 (which is shown in Figure 1), and the longitudinal segments 22a run through wire tubes of the insertion tube 30 and the bending section 40 (as best seen in Figure 7a and 7b). Returning to Figure 5, the longitudinal segments 22a terminate in a distal segment 22b at the interior housing part 70. The distal segment 22b of the steering wire 22 is fixed directly to a wire portion 76 of the interior housing part so that manipulation of the control device causes tensioning of a respective longitudinal segment 22a to articulate the bending section 40 in a distinct direction, thereby allowing the operator to steer the distal tip part 50 during endoscopy.

The wire portion 76 of the interior housing part 70 comprises a channel 76a that accommodates and retain the distal segment 22b of the steering wire 22. The channel 76a extends along an arc about the proximal-distal axis PD. The channel 76a holds the distal segment 22b of the steering wire 22 during assembly.

The interior housing part 70 further comprises a separately formed planar element 80. The planar element 80 contacts, on a distal side, a proximal end 77 of the interior housing part 70, and on an opposite proximal side, the distal end segment 42 of the bending section 40, as shown in Figure 6. Returning to Figure 5, the planar element 80 has two wired through-holes 81 and a single cable hole 82 for the cable 53 (omitted in Figure 5). The steering wire 22 extends through the two through-holes 81. The planar element 80 defines the boundary between the longitudinal segments 22a of the steering wire 22 and the distal segment 22b of the steering wire 22. The planar element 80 consists essentially of a stiffer material than the interior housing part 70. The material of the planar element 80 is preferably a metal and can be formed by punching. The planar element 80 is a plate element and is shaped substantially in the form of a crescent to allow the working channel tube 34 to pass between the horns of crescent shape.

Turning to Figure 6, the wire portion 76 of the interior housing part 70 and the through-holes 81 of the planar element 80 form a first friction-inducing section 76b1 by bending the distal segment 22b of the steering wire 22 around a corner of the through-holes 81 and away from the proximal-distal axis PD by an angle 76c of about 5°. In addition to the channel 76a, the wire portion 76 comprises a pair of corners adjacent to and on opposite sides of the channel 76a that together with the channel 76a form a second friction-inducing section 76b2 by bending the distal segment 22b by an angle of about 80°. The friction-inducing sections 76b1, 76b2 of the wire portion 76 frictionally retain the distal segment 22b of the steering wire 22 in the wire portion 76 and reduce the risk of the steering wire 22 sliding within the wire portion 76.

Turning to Figures 7a and 7b, a method of assembling the exterior housing 60, the camera assembly 51, and the interior housing part 70 is illustrated. First, the exterior housing 60 is positioned upright in a jig so that the proximal opening faces upwards, as shown in Figure 7a and 7b. Then, the camera assembly 51 is inserted through the proximal opening 61 and into the interior cavity 66 of the exterior housing 60 so that the gas volume envelops at least the optical axis 52 of the camera assembly 51 which extends through the window 67 so that the image sensor 54 can capture images through the window 67, as best seen in Figure 3. The interior housing part 70 is then inserted through the proximal opening 61 of the exterior housing 60 so that a substantially uniform gap is formed between the closure surface 72 of distal portion 71 of the interior housing part 70 and the interior surface 65 of the side wall 63 of the exterior housing 60, as shown in Figure 4a (the gap filled with a cured first adhesive is best seen in Figure 8). As best seen in Figure 8, the substantially uniform gap is provided and maintained by guiding protrusions 72a of the closure surface 72 of the interior housing part 70. The guiding protrusions 72a thus maintain a minimum gap distance by contacting the interior surface 65 of the side wall 63 of the exterior housing 60. Then, an injector 100 in the form of a tube is inserted into an injection space 75b formed between legs 75a of the intermediate portion 75, and a first adhesive 110 is injected into this injection space 75b and flows through the proximal opening 61. The first adhesive 110 then fills the substantially uniform gap and is injected, until a level of the first adhesive 110 reaches the proximal opening 61 of the exterior housing 60. The minimum gap distance and the viscosity of the first adhesive 110 is chosen so that capillary forces urge the liquid first adhesive 110 to flow into the gap between closure surface 72 and the interior surface 65. Further, the distal tip part 50 is arranged so that space significantly expands on the distal side of the closure surface 72 (as best seen in Figure 3) so that the liquid first adhesive 110 is kept within the gap until curing and is thus prevented from flowing onwards from the gap and into the interior cavity 66. Then, the first adhesive is cured, thereby adhering the interior housing part 70 to the exterior housing 60 and liquid sealing the gas volume of the interior cavity 66 of the exterior housing 60 to arrive at the arrangement shown in Figure 8, in which the cured first adhesive 111 fills and seals the proximal opening 61.

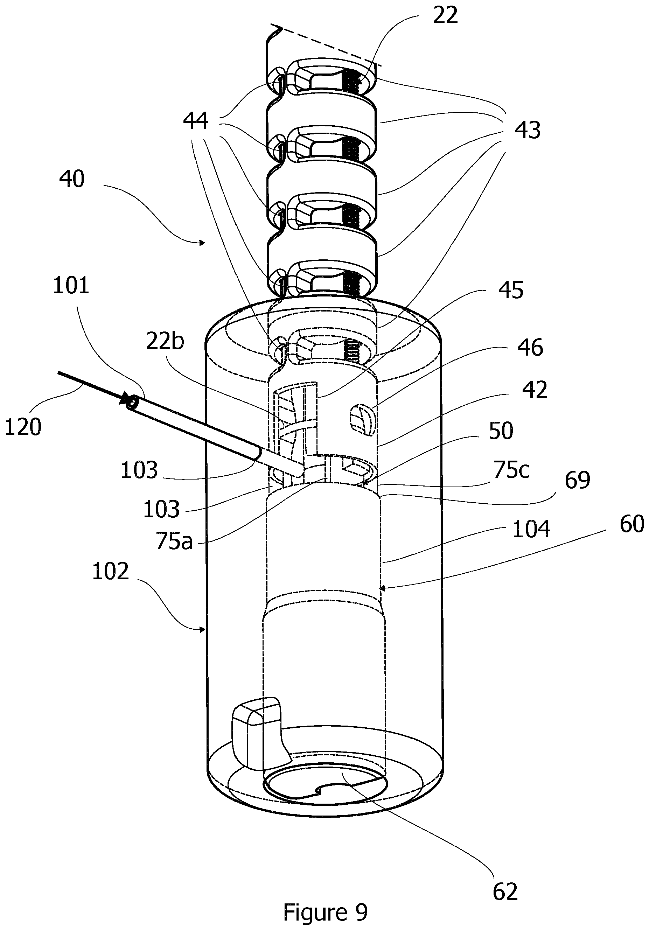

Turning to Figure 9, a method of assembling the interior housing part 70 and the bending section 40 is illustrated. The method may be a continuation of the method described above in connection with Figures 7a, 7b and 8 or may be performed independently. However, this description will assume that the method of Figures 7a, 7b and 8 has been performed. First, an ultraviolet (UV) transparent mould 102 made of silicone is provided. The mould 102 includes sealing portion 104, a mould volume 103 and an exterior injection port 101 providing an injection passage to the mould volume 103. Then, the interior housing part 70 is inserted into the distal end segment 42 of the bending section 40. The radially extending locking protrusions 73a of the interior housing part 70 extend into the proximal-distal cut out 45 of the distal end segment 42 so as to lock the rotation of the interior housing part 70 relative to the distal end segment 42. The interior housing part 70 and the distal end segment 42 are then placed into the mould 102, and the circumference of the sealing portion 104 of the mould 102 expands around the exterior housing 60 upon insertion thereof so that the mould 102 forms a circumferentially extending liquid seal at the proximal end 69 of the exterior housing 60. The mould volume 103 of the mould 102 thus envelops a portion of the interior housing part 70 extending from the proximal opening 61 of the exterior housing 60 to the proximal end 77 of the interior housing part 70. The interior housing part 70 comprises a circumferentially extending gap 75c extending longitudinally between the proximal portion 73 of the interior housing part 70 and the circumferentially extending liquid seal at the proximal end 69 of the exterior housing 60. The intermediate portion 75 of the interior housing part 70 comprises legs 75a bridging the gap 75c between the proximal portion 73 and distal portion 71. The gap 75c thus extends circumferentially around the legs 75a.

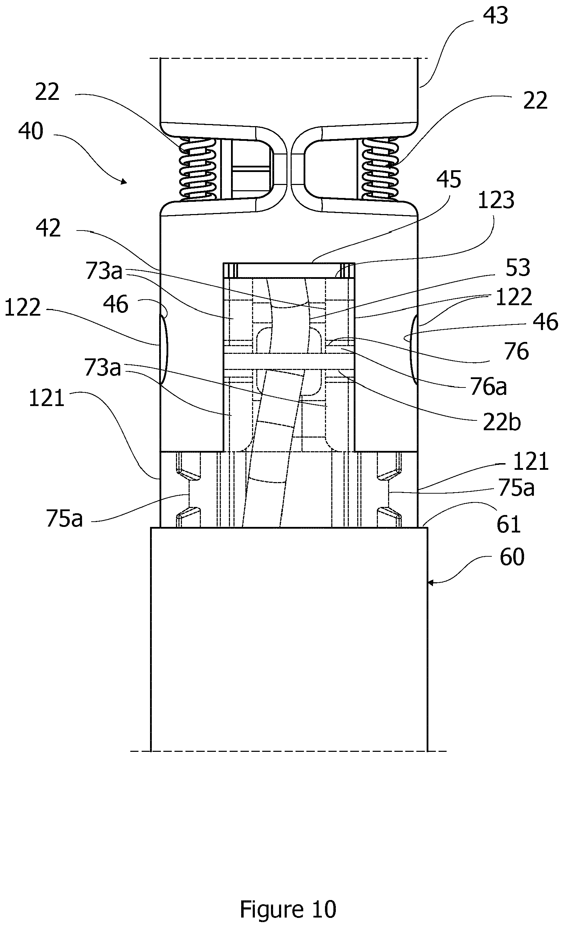

Turning to Figure 10, a second UV-curable adhesive 120 is then injected through the exterior injection port 101 of the mould 102 so that the second adhesive 120 flows into the mould volume 103 of the mould 102 and fills the space between the proximal opening 61 of the exterior housing 60 and the proximal end 77 of the interior housing part 70 including the gap 75c. The second adhesive 120 is injected until it reaches a level 123 at the proximal end 77 of the interior housing part 70. Then, the second adhesive 120 is exposed to UV light via a UV light source (not shown) that is arranged externally of the mould 102 so that the second adhesive 120 cures. The cured section adhesive 120 thus adheres the interior housing part 70 to the distal end segment 42 of the bending section 40 partly by adhering the attachment surface 74 to an interior surface of the distal end segment, partly by forming a cured adhesive anchor 122b fixing the locking protrusions 73a in the cut out 45 of the distal end segment 42, and partly by forming cured adhesive anchors 122 in the anchor portions 46 of the distal end segment. The cured second adhesive 120 further adheres the distal segment 22b of the steering wire 22 to the wire portion 76 of the interior housing part 70 thus fixing the steering wire 22 directly to the interior housing part 70. Further, curing the second adhesive 120 in the gap 75c overmoulds a circumferentially extending cured adhesive surface 121 onto the intermediate portion 75 of the interior housing part 70 between the proximal opening 61 of the exterior housing 60 and the proximal portion 73 of the interior housing part 70. As a last step, a distal portion 91 of the bending cover 90 is placed on the cured adhesive surface 121 so that the exterior surface 92 of the bending cover 90 is substantially flush with the exterior surface 64 of the exterior housing (as indicated in Figure 3) and so that the bending cover covers the bending section, in particular the gaps between hinge members 44 of the bending section 40 as shown in Figure 1.

**LIST OF REFERENCES**

| | | | |
|---|---|---|---|
| 1 | endoscope | 69 | proximal end |
| 11 | monitor | 70 | interior housing part |
| 12 | cable socket | 71 | distal portion |
| 13 | monitor cable | 71b | cable hole |
| 20 | handle | 72 | closure surface |
| 21 | control lever | 72a | guiding protrusion |
| 22 | steering wire | 73 | proximal portion |
| 22a | longitudinal segment | 73a | locking protrusion |
| 22b | distal segment | 73b | cable passage |
| 30 | insertion tube | 74 | attachment surface |
| 31 | proximal end | 75 | intermediate portion |
| 32 | distal end | 75a | leg |
| 34 | working channel tube | 75b | injection space |
| 40 | bending section | 75c | gap |
| 41 | proximal end segment | 76 | wire portion |
| 42 | distal end segment | 76a | channel |
| 42a | outer wall | 76b | friction-inducing section |
| 43 | intermediate segment | 76c | angle |
| 44 | hinge member | 77 | proximal end |
| 45 | proximal-distal cut out | 80 | planar element |
| 46 | anchor portion | 81 | steering wire hole |
| 50 | distal tip part | 82 | cable hole |
| 51 | camera assembly | 83 | working channel cut-out |
| 52 | optical axis | 90 | bending cover |
| 53 | cable | 91 | distal portion |
| 54 | image sensor | 92 | exterior surface |
| 60 | exterior housing | 100 | injector |
| 61 | proximal opening | 101 | exterior injection port |
| 62 | distal end | 102 | mould |
| 63 | circumferentially extending side wall | 103 | mould volume |
| 64 | exterior surface | 104 | sealing portion |
| 65 | interior surface | 110 | first adhesive |
| 66 | interior cavity | 111 | cured first adhesive |
| 67 | window | 120 | second adhesive |
| 68 | working channel passage | 121 | cured adhesive surface |
| 68a | distal opening | 122 | cured adhesive anchor |
| 68b | proximal opening | 123 | level |
| 68c | interior wall | PD | proximal-distal axis |

## Claims

1. An endoscope (1) for visually inspecting inaccessible places, such as human body cavities, the endoscope extending along a proximal-distal axis (PD) from a handle (20) to a distal tip part (50), the endoscope comprising:
- the distal tip part including:
∘ an exterior housing (60) including a circumferentially extending side wall (63) with a proximal opening (61), the circumferentially extending side wall defining an interior cavity (66),
∘ a camera assembly (51) including an image sensor (54), the camera assembly being positioned in the interior cavity of the exterior housing, and
∘ an interior housing part (70) being positioned in the proximal opening of the exterior housing and being a prefabricated component formed separately from the exterior housing, the interior housing part including:
▪ a distal portion (71) with a circumferentially extending closure surface (72) closing the interior cavity of the exterior housing, and
▪ a proximal portion (73) with a circumferentially extending attachment surface (74), the proximal portion being distanced in a proximal direction from the exterior housing;
- a bending section (40) including a proximal end segment (41), a distal end segment (42), and a number of intermediate segments (43) arranged between the proximal end segment and the distal end segment, the distal end segment being attached to the attachment surface of the interior housing part, the bending section being a prefabricated component formed separately from the exterior housing and the interior housing part;
- an insertion tube (30) for insertion into a patient and being attached to the proximal end segment of the bending section;
- the handle for gripping by an operator, the handle being attached to the insertion tube opposite of the bending section, the handle comprising a control device (21); and
- at least one steering wire (22) attached to the control device of the handle and running through the bending section and the insertion tube;
wherein a distal segment (22b) of the at least one steering wire is fixed to a wire portion (76) of the interior housing part so that manipulation of the control device causes bending of the bending section.

2. An endoscope according to claim 1, wherein the bending section is integrally formed as a monolithic component.

3. An endoscope according to any one of the previous claims, wherein the wire portion of the interior housing part comprises a channel (76a) accommodating and retaining the distal segment of the at least one steering wire.

4. An endoscope according to any one of the previous claims, wherein the distal segment of the at least one steering wire passes one or more friction-inducing section(s) (76b) of the wire portion configured to frictionally retain the at least one steering wire in the wire portion.

5. An endoscope according to any one of the previous claims, wherein the distal tip part further comprises a separately formed planar element (80) comprising at least one through-hole (81) and being arranged between the interior housing part and the bending section, wherein the at least one steering wire extends through the at least one through-hole.

6. An endoscope according to claim 5, wherein the wire portion of the interior housing part and/or the at least one through-hole of the planar element is/are configured to bend a distal segment of the at least one steering wire away from the proximal-distal axis by an angle (76c) at the respective through-hole, the angle being at least 2°.

7. An endoscope according to any one of the previous claims, wherein the distal tip part comprises a cured first adhesive (111) adhering the circumferentially extending closure surface to an interior surface (65) of the side wall of the exterior housing, thereby closing the interior cavity of the exterior housing and liquid-sealing the gas volume of the interior cavity.

8. An endoscope according to any one of the previous claims, wherein the distal tip part comprises a cured second adhesive (120) adhering the at least one steering wire to the wire portion of the interior housing part.

9. An endoscope according to any one of the previous claims, wherein the distal end segment of the bending section comprises a circumferentially extending outer wall (42a) with a proximal-distal extending cut-out (45), wherein the proximal portion of the interior housing part comprises one or more locking protrusion(s) (73a) radially extending into the cut-out of the outer wall of the bending section so as to lock the rotation of the interior housing part with respect to the distal end segment of the bending section about the proximal-distal axis.

10. An endoscope according to any one of the previous claims, wherein the interior housing part includes:
- an intermediate portion (75) arranged between and separating the distal portion of the interior housing part and the proximal portion of the interior housing part; and
- a cured adhesive surface (121) extending circumferentially and between the proximal opening of the exterior housing and the proximal portion of the interior housing part;
wherein the endoscope further comprises a bending cover (90) arranged exteriorly of and covering the bending section, a distal portion (91) of bending cover being adhered to the circumferentially extending cured adhesive surface (121) of the interior housing part.

11. A method of assembling a distal tip part for an endoscope according to any one of the previous claims, the method comprising the steps of:
- separately providing:
- an exterior housing including a circumferentially extending side wall with a proximal opening, the circumferentially extending side wall defining an interior cavity,
- a camera assembly including an image sensor,
- an interior housing part including:
- a distal portion with a circumferentially extending closure surface, and
- a proximal portion with a circumferentially extending attachment surface, and
- at least one steering wire having a distal segment;
- attaching the distal segment of the at least one steering wire to a wire portion of the interior housing part; and
- assembling the exterior housing, the camera assembly, and the interior housing part;

12. A method according to claim 11, wherein the step of assembling the exterior housing, the camera assembly, and the interior housing part comprises a first set of steps including:
- positioning the exterior housing upright so that the proximal opening faces upwards;
- inserting the camera assembly through the proximal opening and into the interior cavity of the exterior housing;
- inserting the interior housing part through the proximal opening of the exterior housing so that a substantially uniform gap is formed between the closure surface of distal portion of the interior housing part and an interior surface of the side wall of the exterior housing;
- injecting a first adhesive (110) through the proximal opening so that the first adhesive fills the substantially uniform gap; and
- causing or letting the first adhesive cure, thereby forming a sub-assembly in which the interior housing part is adhered to the exterior housing.

13. A method according to claim 11, wherein the step of assembling the exterior housing, the camera assembly, and the interior housing part further comprises the steps of:
- separately providing:
- a bending section including a proximal end segment, a distal end segment, and a number of intermediate segments arranged between the proximal end segment and the distal end segment, and
- a mould (102) including a mould volume (103), a sealing portion (104) and an exterior injection port (101) providing an injection passage to the mould volume;
- inserting the interior housing part and a distal end segment of the bending section into the mould so that the circumference of the sealing portion of the mould expands around the exterior housing and the sealing portion forms a circumferentially extending liquid seal at a proximal end (69) of the exterior housing, and so that the mould volume of the mould envelops a portion of the interior housing part extending from the proximal opening of the exterior housing to the proximal portion of the interior housing part;
- injecting a second adhesive through the exterior injection port of the mould so that the second adhesive fills the mould volume of the mould; and
- causing or letting the second adhesive cure so that the attachment surface of the interior housing part is adhered to the distal end segment of the bending section and thereby attaching the distal segment of the at least one steering wire to the wire portion of the interior housing part.

14. A method according to claim 12, wherein the step of assembling the exterior housing, the camera assembly, and the interior housing part further comprises a second set of steps including:
- separately providing:
- a bending section including a proximal end segment, a distal end segment, and a number of intermediate segments arranged between the proximal end segment and the distal end segment, and
- a mould (102) including a mould volume (103), a sealing portion (104) and an exterior injection port (101) providing an injection passage to the mould volume;
- inserting the distal end segment of the bending section and the sub-assembly into the mould so that the circumference of the sealing portion of the mould expands around the exterior housing and the sealing portion forms a circumferentially extending liquid seal at a proximal end (69) of the exterior housing, and so that the mould volume of the mould envelops a portion of the interior housing part extending from the proximal opening of the exterior housing to the proximal portion of the interior housing part;
- injecting a second adhesive (120) through the exterior injection port of the mould so that the second adhesive fills the mould volume of the mould; and
- causing or letting the second adhesive cure so that the attachment surface of the interior housing part is adhered to the distal end segment of the bending section and thereby attaching the distal segment of the at least one steering wire to the wire portion of the interior housing part.

15. A method according to any one of claims 13-14, wherein the mould volume comprises, prior to injecting the second adhesive, a circumferentially extending gap (75c), which extends longitudinally between the proximal portion of the interior housing part and the circumferentially extending liquid seal of the mould,
wherein the step of injecting the second adhesive comprises filling said circumferentially extending gap, and the step of cause or letting the second adhesive cure comprises overmoulding, upon curing, a circumferentially extending cured adhesive surface (121) onto the interior housing part between the proximal opening of the exterior housing and the proximal portion of the interior housing part, and
wherein the method further comprises a step of adhering a distal portion (91) of a bending cover (90) to the cured adhesive surface so that an exterior surface (92) of the bending cover is substantially flush with the exterior surface of the exterior housing.
